(19) Europäisches Patentamt
European Patent Office
Office européen des brevets

(11) EP 4 779 015 A1

(12) **EUROPEAN PATENT APPLICATION**
published in accordance with Art. 153(4) EPC

(43) Date of publication:
**22.07.2026 Bulletin 2026/30**

(21) Application number: **24865289.3**

(22) Date of filing: **30.08.2024**

(51) International Patent Classification (IPC):
***C12N 5/071*** *(2010.01)*

(52) Cooperative Patent Classification (CPC):
**C12N 5/06**

(86) International application number:
**PCT/JP2024/031255**

(87) International publication number:
**WO 2025/057787 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.09.2023 JP 2023149357**

(71) Applicant: **Toyobo Co., Ltd.**
**Kita-ku**
**Osaka-shi, Osaka 530-0001 (JP)**

(72) Inventors:
- **INOUE, Keita**
  **Otsu-shi, Shiga 520-0292 (JP)**
- **UMEDA, Ayano**
  **Otsu-shi, Shiga 520-0292 (JP)**
- **KOBAYASHI, Miduki**
  **Otsu-shi, Shiga 520-0292 (JP)**
- **KAWAKAMI, Yoshitaka**
  **Otsu-shi, Shiga 520-0292 (JP)**

(74) Representative: **Vossius & Partner**
**Patentanwälte Rechtsanwälte mbB**
**Siebertstraße 3**
**81675 München (DE)**

(54) **METHOD FOR PRODUCING EXTRACELLULAR VESICLES**

(57) The objective of the present invention is to provide a method for separating and purifying extracellular vesicles with a high recovery rate and a low cost. The present invention relates to a method for producing extracellular vesicles, the method comprising Step (a) of preparing a biological sample comprising the extracellular vesicle, Step (b) of adjusting an electrical conductivity of the biological sample to 4 mS/cm or more and 15 mS/cm or less, Step (c) of contacting the biological sample with a positively charged support, and Step (d) of collecting the extracellular vesicle by contacting the support with a buffer solution comprising a metal salt.

EP 4 779 015 A1

## Description

TECHNICAL FIELD

The present invention relates to a method for producing extracellular vesicles with a high recovery rate.

BACKGROUND ART

Extracellular vesicles are a collective term for vesicles that are secreted by almost all living cells and have a heterogeneous lipid bilayer structure. Extracellular vesicles are mainly classified into exosomes, microvesicles and apoptotic bodies depending on the biogenesis mechanism thereof.

An exosome is formed by inward budding of a late endosome membrane and becoming a fully matured vesicle upon fusion with a plasma membrane, and is secreted into the extracellular space via exocytosis. Exosomes are extracellular vesicles of approximately 30 to 150 nm in diameter that is released from a eukaryotic cell such as those of an animal, a plant and a fungus. It has been known that an exosome exhibits important functions in intercellular communication. For example, an exosome encapsulates a protein, an mRNA, a miRNA or the like and propagates them in a body fluid. In recent years, an exosome has been suggested to be involved in promoting cancer metastasis and exacerbating tumor progression. Thus, a diagnostic marker focusing on an exosome has been developed, such as those that use a miRNA expressed in a tumor-derived exosome as a tumor marker to predict cancer progression and to judge metastasis and clinical prognosis of cancer.

An exosome isolated and purified from a biological source is expected to be applied to a drug delivery system (DDS) as a bio-derived carrier that encapsulates an intracellular nucleic acid, a protein or the like. Thus, there is a growing demand for a technology that enables the high-purity isolation and purification of an exosome.

A microvesicle is generated through outward budding of a plasma membrane followed by its detachment. A microvesicle varies widely in size, with the diameter ranging from approximately 100 to 1,000 nm. An apoptotic body is generated during apoptosis of a cell, and the size thereof varies widely, typically ranging from 50 to 5,000 nm in diameter. A microvesicle and an apoptotic body are also expected to have applications similar to those of an exosome.

An example of a method for isolating and purifying extracellular vesicles includes differential centrifugation, ultracentrifugation method such density-gradient centrifugation, a precipitation method employing a polymer such as polyethylene glycol, and a chromatography method such as size exclusion chromatography and affinity chromatography. An ultracentrifugation method is exemplified as a general method, and high purity extracellular vesicles may be obtained by density-gradient centrifugation in some cases. For example, Patent document 1 discloses a method for producing an exosome by subjecting a biological sample to ultrafiltration and then anion-exchange column chromatography.

PRIOR ART DOCUMENT

PATENT DOCUMENT

Patent document 1: WO 2020/027185

DISCLOSURE OF THE INVENTION

PROBLEMS TO BE SOLVED BY THE INVENTION

Ultracentrifugation method has problems such as the decrease in quality due to the structural change of extracellular vesicles or destruction during a centrifugation process and a low recovery rate. A method using anion-exchange column chromatography has a problem that the number of adsorbed extracellular vesicles per unit volume is insufficient.

The objective of the present invention is to provide a method for separating and purifying extracellular vesicles with a high recovery rate and a low cost, and a method for producing extracellular vesicles by the purification method.

MEANS FOR SOLVING THE PROBLEMS

The inventors of the present invention conducted extensive research into the preparation of extracellular vesicles. As a result, the inventors found that the target extracellular vesicles can be recovered with high purity and a high recovery rate by optimizing the steps to culture a cell, remove an impurity, and separate and recover extracellular vesicles.

In other words, the inventors completed the present invention by finding that the above-described problem can be

solved by the following method.

[1] A method for producing extracellular vesicles, the method comprising:

Step (a) of preparing a biological sample comprising the extracellular vesicles,
Step (b) of adjusting an electrical conductivity of the biological sample to 4 mS/cm or more and 15 mS/cm or less,
Step (c) of contacting the biological sample with a positively charged support, and
Step (d) of collecting the extracellular vesicles by contacting the support with a buffer solution comprising a metal salt.

[2] The method according to the above [1], wherein a cell is isolated from an animal or a frozen cell from a cell stock is thawed, and subsequently the cell or the thawed cell is cultured in the Step (a).
[3] The method according to the above [1] or [2], comprising a step of removing all or a part of an impurity from the biological sample between the Step (a) and the Step (b).
[4] The method according to the above [3], wherein 1 or more selected from the group consisting of centrifugation, precision filtration, ultrafiltration and dialysis are carried out in the step of removing all or a part of the impurity.
[5] The method according to any one of the above [1] to [4], wherein the support is an anion-exchange separation membrane in the Step (c).
[6] The method according to any one of the above [1] to [5], wherein the support has a tertiary amine and/or a quaternary ammonium group in the Step (c).
[7] The method according to any one of the above [1] to [6], comprising a step of washing the support by contacting the support with a buffer solution between the Step (c) and the Step (d).
[8] The method according to any one of the above [1] to [7], wherein the buffer solution comprising the metal salt is any one of Tris-HCl buffer solution, phosphate-buffered saline solution and Tris-buffered saline solution in the Step (d).
[9] The method according to any one of the above [1] to [8], wherein the metal salt is NaCl in the Step (d).
[10] The method according to any one of the above [1] to [9], wherein an electrical conductivity of a sample comprising the collected extracellular vesicles is adjusted to more than 15 mS/cm in the Step (d).
[11] The method according to any one of the above [1] to [10], wherein the extracellular vesicles are exosomes and/or microvesicles.

## EFFECT OF THE INVENTION

The amount of target extracellular vesicles adsorbed on a support can be maximized by adjusting the electrical conductivity of a biological sample and then contacting the biological sample with a positively charged support in the method for purifying extracellular vesicles from a biological sample according to the present invention. In addition, it is possible to prepare the group of a particle that strongly expresses an extracellular vesicle marker per the particle.

## BRIEF DESCRIPTION OF THE DRAWINGS

[Figure 1] Figure 1 is a schematic diagram to demonstrate one example of a culture apparatus to prepare a biological sample containing extracellular vesicles.
[Figure 2] Figure 2 is the graph as a calibration curve to show the relation of a salt concentration and electrical conductivity.
[Figure 3] Figure 3 is the photograph to show the result of Western blotting.

## MODE FOR CARRYING OUT THE INVENTION

The present invention relates to a novel method for purifying and producing extracellular vesicles such as exosomes from a biological sample. The present invention is hereinafter described but is not restricted to the following specific examples. The numerical range, "x to y", includes both of x and y in this disclosure.

### 1. Step to prepare biological sample

A biological sample containing target extracellular vesicles is prepared in this step.
A biological sample is not particularly restricted as long as the biological sample contains extracellular vesicles to be purified and produced, and is preferably liquid. An example of a biological sample specifically includes a cell culture

fluid, a culture supernatant separated from a cell culture fluid, interstitial fluid, blood, serum and blood plasma separated from blood, urine, spinal fluid, lymph, ascitic fluid and milk. When a biological sample is not liquid, the biological sample is preferably dissolved or dispersed in liquid such as a buffer solution.

A method for purifying extracellular vesicles from a biological sample in the present invention preferably comprises the steps of preparing the biological sample comprising the extracellular vesicles, adjusting an electrical conductivity of the biological sample, contacting the biological sample with a positively charged support, washing the positively charged support with a buffer solution, and obtaining the extracellular vesicles by contacting the positively charged support with a buffer solution comprising a metal salt.

Extracellular vesicles are a general term for a small vesicle that is secreted by almost all living cells and has a heterogeneous lipid bilayer structure. Extracellular vesicles are roughly classified into 3 kinds of exosomes, microvesicles and apoptotic bodies on the basis of the intracellular production mechanism. The diameter of an exosome is 30 to 150 nm. An exosome is formed by inward budding of a late endosome membrane and becoming a fully matured vesicle upon fusion with a plasma membrane, and is secreted into the extracellular space via exocytosis. On the one hand, a microvesicle is generated through outward budding of a plasma membrane followed by its detachment. The diameter thereof is 100 nm to 1,000 nm. An apoptotic body is generated during organizational apoptosis of a cell, and the size thereof is 50 nm to 5,000 nm in diameter.

The "extracellular vesicles" may be extracellular vesicles contained in a biological sample or extracellular vesicles produced by a target cultured cell in the present invention, and the size and the figure of extracellular vesicles is not particularly restricted. The extracellular vesicles may be any one of a natural and an unnatural (artificial) extracellular vesicles.

For example, extracellular vesicles can be prepared from a cultured cell. The "cultured cell" means a general cell cultured in vitro (out of a living body) and is not restricted to a specific commercially established cell line in the present invention. A cell contained in a culture material such as a primary culture prepared by culturing a cell, a tissue and an organ derived from a human or an organism other than a human, particularly a living mammal, is included in the "cultured cell". A cell of a commercially established cell line of various culture tumor cells is a typical example of the "cultured cell". The "tumor cell" such as a "cancer cell" means a cell that forms various tumors and is typically a cell that abnormally grows, independently of a surrounding normal tissue, and an example thereof includes a cancerous cell. A preferable example of the "cultured cell" includes a "cultured tumor cell" derived from a cell that constitutes various cell tumors such as lymphomas and melanoma. An example of the cultivated tumor cell includes cultured neuroblast and retinoblast.

A cell culture material contains a target cultured cell and a component contained in the culture medium of the cultured cell. An example of a cell to be cultured includes a cell line that is generally available, typically commercially available, such as a tumor cell line derived from a human or a mammal other than a human. For example, a cultured tumor cell derived from uterus cancer, ovarian cancer, breast cancer or the like can be used, and HEK293 cell derived from human embryonic kidney can be used.

The component of a culture medium to culture a cell may follow conventionally recommended medium formulations depending on the type of the culture cell and is not particularly restricted in carrying out the present invention. For example, a medium prepared by supplementing commercially available media such as $\alpha$-MEM, DMEM and MEM with serum such as FBS: fetal bovine serum and, as necessary, a component such as antibiotics can be used. It is preferred in this disclosure to use a medium containing serum in which extracellular vesicles derived from a serum-donor animal are not mixed in terms of the recovery of high purity extracellular vesicles.

When extracellular vesicles are purified from a culture supernatant in the present invention, a cell to be cultured may be prepared by thawing a frozen cell stored as a cell stock. Alternatively, a cell to be cultured may be prepared by purifying the cell from a tissue of an animal such as a human in another embodiment. An example of a cell includes a cell in blood and a stem cell. A medium used for the culture may be any one of a serum-free medium or a serum-supplemented medium. An example of the cell in blood includes T cell. An example of a stem cell includes ES cell, iPS cell and mesenchymal stem cell.

A culture method is not particularly restricted, and an example thereof includes plane culture using a culture flask, a dish or a plate; microcarrier culture, in which a cell is cultured by suspending a bead-shaped microcarrier to which the cell is adhered in a culture medium; and culture using a hollow fiber membrane or a hollow gel.

The culture period is not particularly restricted, and it is preferred to culture a cell in the temperature condition of about 37°C under a $CO_2$-rich atmosphere for about 2 to 5 days in terms of the efficient recovery of a sufficient amount of extracellular vesicles. For example, the $CO_2$ concentration may be adjusted to about $5\pm2\%$.

Figure 1 shows one example of the configuration of a hollow fiber membrane-type cell culture apparatus. In Figure 1, 1 and 2 are respectively a liquid culture medium storage container having gas permeability, such as a culture bag. A flow path connects the liquid culture medium storage container 1 to the cell culture vessel 17 through the aseptic connector 13 and the valve 4, and the culture medium in the liquid culture medium storage container 1 is supplied into the hollow part of the hollow fiber membrane. In addition, a flow path connects the liquid culture medium storage container 2 to the cell culture vessel 17 through the aseptic connector 14 and the valve 5, and the culture medium in the liquid culture

medium storage container 2 is supplied into the outside of the hollow fiber membrane.

A flow path connects the hollow part of the cell culture vessel 17 to the waste liquid collection container 18 through the valve 7, the valve 11, the liquid feed pump 20, the valve 12 and the aseptic connector 16 to discard the culture medium that has passed through the cell culture vessel 17. The supply of the liquid such as a culture medium from the liquid culture medium storage container 1 to the cell culture vessel 17, the discharge of the liquid from the cell culture vessel, and the flow rate of the liquid to be discharged can be controlled by the liquid feed pump 20 provided in the flow path. In addition, a flow path connects the outside of the cell culture vessel 17 to the waste liquid collection container 18 through the valve 6, the valve 9, the liquid feed pump 19, the valve 10 and the aseptic connector 16 to discharge the culture medium that has passed through the cell culture vessel 17. The supply of the liquid such as a culture medium from the liquid culture medium storage container 2 to the cell culture vessel 17, the discharge of the liquid from the cell culture vessel, and the flow rate of the liquid to be discharged can be controlled by the liquid feed pump 19 provided in the flow path.

In Figure 1, the cell collection container 3 is connected to the hollow part of the cell culture vessel 17 through the valves 7 and 8 and the aseptic connector 15. If necessary, the cell may be collected by additionally providing a pump or the like in the flow path. The cell may be collected by removing the cell culture vessel 17 from the cell culture apparatus. The configuration from the branch of the waste system flow path on the lumen side to the cell collection container 3 may be omitted in such a case.

In Figure 1, the $CO_2$ incubator 31 preferably covers most of the above-described configuration; however, as long as at least the gas-permeable liquid culture medium storage containers 1 and 2 and the cell culture vessel 17 are covered, the other components may be disposed outside the $CO_2$ incubator 31.

The operation controller 21 (operation panel) may be provided to perform the above-described operations and processes and to carry out monitoring in Figure 1.

An impurity such as unnecessary large cell debris may be removed in the step to prepare a biological sample containing extracellular vesicles in the present invention. An example of the means for removing an impurity includes centrifugation, precision filtration, ultrafiltration and dialysis. For example, centrifugation is carried out at 4°C and 10,000×g for 15 to 30 minutes. The supernatant can be obtained by centrifugation to remove cell debris or the like. Additional centrifugation may be carried out to further remove an impurity. When additional centrifugation is carried out, centrifugal acceleration (g) may be adjusted to be higher to remove cell debris of a medium size or smaller. When the purification target is an exosome or a microvesicle, an apoptotic body may be also removed.

Precision filtration is a treatment using a membrane filter having a pore size of 0.2 to 0.5 μm, and cell debris of a medium size or the like may be removed similarly to centrifugation by collecting the filtrate.

The supernatant obtained by centrifugation and/or the filtrate obtained by precision filtration may be treated by ultrafiltration using a membrane filter having a pore size of 0.1 μm or less, preferably 0.05 μm or less, and/or dialysis. A smaller impurity than extracellular vesicles can be removed and extracellular vesicles can be concentrated by ultrafiltration and/or dialysis.

Ultracentrifugation may be carried out in the step to prepare a biological sample containing extracellular vesicles in the present invention. A smaller impurity than extracellular vesicles can be removed and extracellular vesicles can be concentrated by ultracentrifugation to apply centrifugal force of 100,000×g or more and thus precipitate extracellular vesicles.

2. Step to adjust electrical conductivity

The electrical conductivity of the biological sample prepared in the above-described Step 1 is adjusted to 4 mS/cm or more and 15 mS/cm or less in this step. The electrical conductivity is preferably adjusted by adjusting a salt concentration. In general, the biological sample has an osmotic pressure close to that of physiological saline, and the osmotic pressure facilitates cell viability. The concentration and the osmotic pressure of physiological saline are respectively 0.154 mmol/L and 308 mOsm/L. The osmotic pressure of the biological sample is largely attributable to an ionic component present in the liquid, and thus a positive correlation is observed between the electrical conductivity and the salt concentration as well as between the electrical conductivity and the osmotic pressure of the biological sample. When the electrical conductivity is adjusted to 15 mS/cm or less, the adsorption efficiency of extracellular vesicles on the positively charged support can be enhanced, since extracellular vesicles have a relatively weak negative charge. On the one hand, when the electrical conductivity is adjusted to 4 mS/cm or more, the degradation of extracellular vesicles can be suppressed. The electrical conductivity is preferably 9 mS/cm or more and 14 mS/cm or less. When the electrical conductivity is 15 mS/cm or less, the concentration of a negative charge ion such as a chloride ion among the buffer solution component such as a salt in the biological sample is decreased, the proportion of the ion-exchange group in the support that is ionically bound to a negatively charged ion such as a chloride ion is decreased, and thus the number of the ion-exchange group effective for adsorbing negatively charged extracellular vesicles is increased. An increase in the density of the effective ion-exchange group present on the surface of the support generates a zeta potential capable of attracting extracellular vesicles, which has a lower diffusion coefficient than a low-molecular-weight ion, toward the support

surface. As a result, a particle with a smaller magnitude of negative charge among extracellular vesicles is particularly more readily adsorbed onto the support. On the one hand, when the electrical conductivity is 4 mS/cm or more, the osmotic pressure of the aqueous solution component in the sample does not decrease excessively, thereby suppressing the damage to extracellular vesicles caused by an osmotic pressure difference generated between the inside and the outside of the lipid bilayer membrane of the extracellular vesicle. Thus, it is important to adjust the electrical conductivity within the above-described appropriate range. When the electrical conductivity is 9 mS/cm or more and 14 mS/cm or less, the NaCl concentration in an NaCl aqueous solution such as 10 mM Tris-HCl is converted to 79 mmol/L or more and 130 mmol/L or less.

A method for decreasing the electrical conductivity or the salt concentration of the biological sample containing extracellular vesicles is not particularly restricted, and the biological sample may be diluted with a buffer solution having the lower electrical conductivity or the lower salt concentration than those of the biological sample. An example of the buffer solution to be used includes Tris-HCl buffer (Tris-HCl) having the concentration of about 10 mM, phosphate-buffered saline solution (PBS) and Tris-buffered saline solution (TBS) in such a case. The electrical conductivity and the salt concentration of the biological sample containing extracellular vesicles can be adjusted by the substitution of the buffer solution with ultrafiltration and the desalination with dialysis as other embodiments.

3. Step to contact with support

The biological sample of which electrical conductivity is adjusted in the above-described Step 2 is contacted with a positively charged support in this step. The support is not particularly restricted in this step, and an example thereof preferably includes a polysulfone resin and a cellulosic resin such as cellulose acetate and cellulose triacetate. The support may be any one of a sheet separation membrane such as a flat-sheet membrane, a hollow fiber separation membrane (hollow fiber membrane), a porous particle and a non-porous particle.

A method for imparting a positive charge to the support is not particularly restricted in the present invention, and an example of the method includes a method in which a cationic compound is applied after molding of the support. An example of the method more specifically includes a method for imparting a positive charge to the support having a hydroxy group by contacting the support with glycidyltrimethylammonium or glycidyldiethylamine. In addition, a column may be filled with a commercially available diethylaminoethyl (DEAE) cellulose resin to be used. DEAE cellulose means an anion exchanger prepared by introducing a diethylaminoethyl group into cellulose and can be obtained from a supplier such as Sigma-Aldrich.

A cellulosic ion-exchange membrane prepared by substituting at least a part of a hydroxy group or an acetyl group at the 2-position, 3-position and 6-position of a porous base membrane comprising a cellulosic polymer with a positively charged compound is preferably used as a positively charged support in the present invention.

A porous base membrane comprising a cellulosic polymer can be produced by a wet-process method. The wet-process method means a method in which a cellulosic polymer is mixed with a solvent and a non-solvent to prepare a membrane-forming solution and then the membrane-forming solution is extruded into a hollow form and introduced into a coagulation bath to cause phase separation. A dry-process method, a thermally induced phase separation method, a stretching method or the like may be also used. A flat-sheet porous base membrane can be prepared by dissolving a cellulosic polymer in a solvent, uniformly applying the solution on a base material such as glass to be immersed in a coagulation liquid for coagulation, and then washing and drying it if necessary.

The solvent to prepare the membrane-forming solution is preferably a polar solvent such as γ-butyrolactone, N-methylpyrrolidone and dimethylacetamide. One of the solvents may be used alone, or a plurality of the solvents may be mixed to be used. If necessary, a non-solvent such as water, glycerol, ethylene glycol, triethylene glycol, polyethylene glycol 200 and polyethylene glycol 400 may be added. The mixing ratio of the solvent and the non-solvent in the membrane-forming solution, i.e. solvent/non-solvent ratio, is preferably 90/10 to 10/90.

An example of the coagulation liquid preferably includes a mixed liquid of a solvent and a non-solvent such as water. The solvent/non-solvent ratio in the coagulation liquid is preferably adjusted to match the solvent/non-solvent ratio in the membrane-forming solution. The variation in the composition of the coagulation liquid even during continuous membrane formation can be suppressed by matching the solvent/non-solvent ratios of the membrane-forming solution and the coagulation liquid. The weight ratio of N-methylpyrrolidone : water in a mixed solution of N-methylpyrrolidone and water is preferably within the range of 30:70 to 50:50 in the case of a flat-sheet membrane. When the coagulation bath is adjusted to have such a composition, the pore diameter of the membrane surface that first comes into contact with the coagulation bath becomes larger than that of the membrane surface on the side in contact with the base material and thus an asymmetric structure is formed in the thickness direction. When the concentration of N-methylpyrrolidone in the coagulation bath is 25 mass% or more and less than 30 mass%, the pore diameter of the membrane surface that first comes into contact with the coagulation bath becomes equal to that of the membrane surface on the side in contact with the base material and thus the asymmetric structure of the membrane tends to be relaxed. In addition, when the concentration of N-methylpyrrolidone in the coagulation bath is less than 25 mass%, a dense layer substantially free of distinct pores

tends to be formed on the membrane surface that first contacts the coagulation bath. The temperature of the coagulation liquid is preferably 5 to 60°C.

The produced porous base material membrane is washed with hot water to remove the excessive solvent or the like. The washed porous base material membrane may be stored in water. Alternatively, the pores of the membrane may be filled with a pore-holding agent such as an aqueous glycerol solution and then dried.

Next, an ion-exchange group is introduced into the produced porous base material membrane. Specifically, a hydroxy group is subjected to a charge-imparting treatment after the deacetylation treatment. An example of a method for producing a cationic cellulose membrane as the ion-exchange membrane by introducing a positively charged group to a hydroxy group of a cellulosic porous base material membrane includes a production method by immersing the cellulosic porous base material membrane in a non-solvent in the presence of an alkali, adding a solution of a positively charged compound dropwise, performing the reaction in a heating condition, and then quenching the reaction mixture with an alcohol.

The above-described non-solvent is not particularly restricted as long as a cellulosic porous base material membrane cannot be dissolved in the non-solvent and a positively charged compound can be dissolved in the non-solvent, and an example thereof preferably includes water, tetrahydrofuran (THF), dimethylsulfoxide (DMSO) and dimethylformamide (DMF). One of the solvents may be used alone or a plurality of the solvents may be mixed to be used depending on the solubility of the raw material.

An example of the alkali used for the deacetylation includes a hydroxide such as lithium hydroxide, potassium hydroxide, sodium hydroxide and cesium hydroxide; a carbonate salt; and an organic amine. Industrially inexpensive sodium hydroxide is preferably used among them. The concentration of the alkali to be used is preferably adjusted within the range of 0.05 mass% to 5.0 mass%. When the concentration of the alkali is 0.05 mass% or more, the amount of the introduced charged group may be increased and thus the amount of an adsorbed extracellular vesicle per unit volume can be increased more reliably. When the concentration of the alkali is 5.0 mass% or less, the swelling degree of the membrane can be suppressed and thus the structure such as the pore diameter and the physical properties such as the membrane strength of an ion-exchange membrane may be more reliably maintained even after the introduction of a charged group. The concentration of the alkali is more preferably 0.1 to 2.5 mass% and even more preferably 0.2 to 2.0 mass%.

The amount of a charged group added to the cellulose membrane can be controlled by the amount of the added positively charged compound. For example, when an epoxy group-containing tertiary amine compound is used, the concentration of the tertiary amine compound in a non-solvent is preferably included within the range of 0.01 mol/L to 3.0 mol/L. The ion exchange capacity in such a case per the weight of the cellulose membrane corresponds to 0.05 meq/g to 0.9 meq/g. When the epoxy group-containing quaternary ammonium compound is used, the concentration of the quaternary ammonium compound in a non-solvent is preferably included within the range of 0.01 mol/L to 1.0 mol/L. The ion exchange capacity in such a case per the weight of the cellulose membrane corresponds to 0.05 meq/g to 0.4 meq/g. When the amount of the added positively charged group is excessively large, the membrane strength in water may become low in some cases due to large amount of the added positively charged group per one polymer molecule and high water solubility. When the amount of the added positively charged group is excessively small, an adsorption efficiency of extracellular vesicles may become low in some cases.

The positively charged support such as an ion-exchange membrane is preferably installed in a vessel provided with an inlet for introducing a liquid to be treated and an outlet for discharging a biological sample that has undergone an ion exchange treatment in the form of a device in the present invention. Such a device has a first chamber and a second chamber separated by an ion-exchange membrane, wherein a biological sample as a liquid to be treated introduced into the first chamber passes through the ion-exchange membrane and moves into the second chamber. Extracellular vesicles in the biological sample as the treated liquid are adsorbed onto the surface and the pore surface of the ion-exchange membrane on this occasion.

The ion-exchange membrane as a positively charged support may be a cellulosic ion-exchange membrane prepared by substituting all or a part of a hydroxy group or an acetyl group at the 2-position, the 3-position and the 6-position of a cellulosic polymer with a positively charged compound in the present invention.

An example of a cellulosic polymer includes cellulose acetate, cellulose propionate, cellulose butyrate, cellulose acetate propionate, cellulose acetate butyrate, cellulose acetate laurate, cellulose acetate oleate and cellulose acetate stearate in the present invention, and cellulose acetate is preferably used in terms of the ease of introduction of a positively charged compound. Cellulose acetate is commercially available in various forms with different degrees of acetylation and molecular weight, and cellulose acetate and/or cellulose triacetate with an acetylation degree of approximately 52 to 62 is more preferably used.

The tertiary amine represented by the following formula (I) or the quaternary ammonium compound represented by the following formula (II) is preferably used as a positively charged compound in the present invention. In the formulas, $R^1$ to $R^3$ may be the same as or different from each other and are a hydrogen atom or a linear or branched $C_{1-10}$ alkyl group, n attached to the methylene group is an integer of 1 to 5, x is 1 or more selected from the group consisting of a halogen atom such as fluoro, chloro, bromo and iodo, a leaving group such as tosylate, triflate and mesylate, a silyl group such as alkoxy

silyl and silanol, an epoxide group, an isocyanate group and a carboxy group. The substituent group X of the positively charged compound is not restricted, and the compound of which X is chloro, a silyl group or an epoxide group is preferred in terms of the ease of obtaining the raw material thereof.

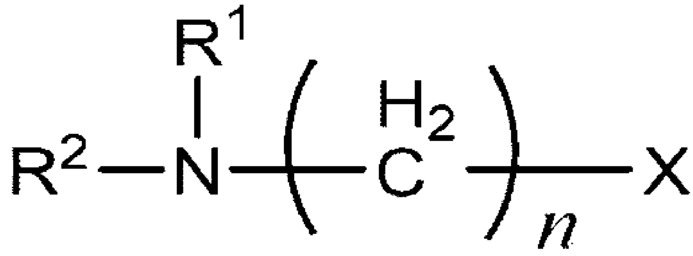

(I)

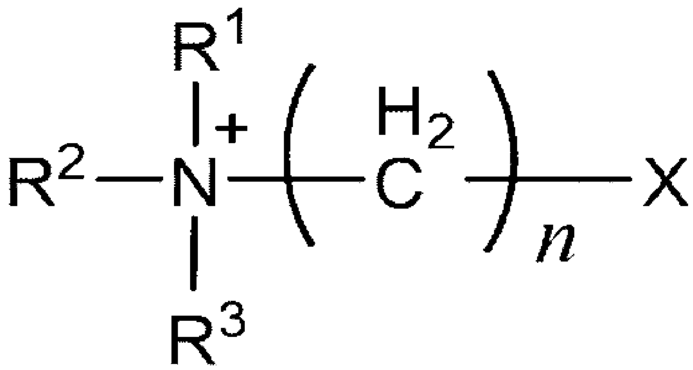

(II)

The cellulosic ion-exchange membrane may be any one of a so-called homogeneous membrane and a so-called asymmetric membrane in the present invention. The pore size is approximately uniform from one surface toward the other surface of a homogeneous membrane. The pore size changes continuously or discontinuously from one surface to the other surface of an asymmetric membrane. The minimum pore diameter of the ion-exchange membrane is preferably 50 nm or more and 1000 nm or less. An asymmetric membrane preferably has a minimum pore diameter layer in the vicinity of either surface. When the minimum pore diameter is large, the probability of the contact of extracellular vesicles such as an exosome with the membrane surface and/or the pore surface may be decreased and thus the adsorption rate of extracellular vesicles in the liquid to be treated, such as a biological sample, may be decreased in some cases. In addition, cell debris or the like may not be removed in some cases. When the minimum pore diameter is small, the recovery rate of extracellular vesicles that has entered inside the membrane may be decreased or it may take longer to recover extracellular vesicles in some cases. The particle diameter of a polystyrene particle of which rejection rate measured using a polystyrene particle dispersion is 80% or more is determined as a minimum pore diameter.

The average particle diameter on the surface of the ion-exchange membrane, such as the surface of the side without the minimum pore size layer, is preferably 100 nm or more and 5000 nm or less. When the average pore diameter on the surface is 100 nm or more, a biological sample can be treated at a sufficiently fast rate. When the average pore diameter on the surface is 5000 nm or less, the adsorption of extracellular vesicles onto the support can be sufficiently promoted to maintain the recovery rate. When the average pore diameter on the surface is included within the above-described range, the effect of depth filtration can be exerted more surely. The average diameter on the surface can be calculated by an image processing software, "Image J", on the basis of 1,000 to 20,000-power photographs of both membrane surfaces using a scanning electron microscope (SEM).

A biological sample as a liquid to be treated may be processed by either cross-flow filtration or dead-end filtration. When an asymmetric membrane is used, a biological sample as a liquid to be treated may be supplied to the side having a large pore diameter or the side having a small pore diameter. For example, the surface having a large pore diameter is used as a primary side and the surface having a small pore diameter is used as a secondary side in the case of a biological sample such as a culture supernatant, of which solid component concentration is low and an extracellular vesicle concentration is high; as a result, when a biological sample is supplied to the membrane to adsorb extracellular vesicles on the membrane by charge, the clogging of the membrane pore caused by the adsorbed extracellular vesicle can be inhibited. On the one hand, the surface having a small pore diameter is used as a primary side and the surface having a large pore diameter is used as a secondary side in the case of a biological sample such as blood serum, blood plasma, urine and milk, of which solid component concentration is relatively higher than an extracellular vesicle concentration; as a result, an impurity can be removed through surface filtration and the clogging of the membrane caused by the complete clogging of pores can be inhibited.

The ion exchange capacity of the positively charged support is preferably 0.05 meq/g or more and 1.5 meq/g or less in the present invention. When the ion exchange capacity is 0.05 meq/g or more, the zeta potential of the membrane becomes stable and thus a target substance to be purified having a small diffusion coefficient, such as extracellular vesicles, can be sufficiently drawn to the membrane and a large capacity device is not needed due to a large adsorption amount. When the ion exchange capacity is 1.5 meq/g or less, the strength of the membrane is sufficient, and the adsorption amount of the membrane per the unit volume becomes appropriate and thus membrane clogging can be suppressed. When a tertiary amine compound is used, the ion exchange capacity per the support mass is preferably 0.03 meq/g or more and 0.9 meq/g or less. When a quaternary ammonium compound is used, the ion exchange capacity per the support mass is preferably 0.03 meq/g or more and 0.4 meq/g or less.

The form of the support may be a flat-sheet membrane or a hollow fiber membrane, and the thickness of the membrane is preferably 10 μm to 1000 μm in the present invention. When the thickness of the membrane is 10 μm or more, the strength of the membrane is sufficiently high and thus the handling convenience during membrane formation and device fabrication is excellent. When the thickness of the membrane is 1000 μm or less, the time to recover extracellular vesicles can be shorten and thus the recovery rate can be maintained. The inner diameter of the hollow fiber membrane is preferably 50 μm to 1000 μm. When the inner diameter is 50 μm or more, the shear stress of the liquid flowing in the hollow portion is sufficiently small and thus the damage to extracellular vesicles can be sufficiently suppressed. When the inner diameter is 1000 μm or less, the membrane area per one device can sufficiently become large.

The zeta potential of the support in the condition of pH 6.5 to 7.5 is preferably included within the range of 10 mV to 70 mV in the present invention. When the zeta potential is 10 mV or more, the adsorption force to extracellular vesicles having a low diffusion coefficient is sufficiently strong and thus the adsorption property can be ensured. When the zeta potential is 70 mV or less, the membrane strength is sufficiently high, and the adsorption amount per a unit volume of the membrane becomes appropriate and thus membrane clogging can be suppressed in the case where a purification target is a particle such as extracellular vesicles. The zeta potential of the sheet-shaped support can be measured using a flat plate zeta potential measurement cell.

The total specific surface area of the pores having the pore diameter of 30 nm or more is preferably 3 $m^2$/g or more and more preferably 8 $m^2$/g or more with respect to the specific surface area of the support in the present invention. When the specific surface area 3 $m^2$/g or more, a sufficient amount of an adsorbed extracellular vesicle may be ensured. The specific surface area is generally measured using a gas adsorption method and mercury intrusion porosimetry. The specific surface area of micropores of 2 nm or less and mesopores of 2 nm to 50 nm can be detected by a gas adsorption method, and mesopores and macropores of 50 nm or more can be detected by mercury intrusion porosimetry. Though the larger specific surface area is preferred in order to ensure a sufficient adsorption capacity for extracellular vesicles, it is needed that the specific surface area constituted by mesopores and macropores having the pore diameter of 30 nm or more be sufficiently large in order to increase the adsorption amount of extracellular vesicles per unit volume of the membrane, since extracellular vesicles cannot enter pores having the pore diameter of less than 30 nm. Though an excessively large specific surface area of the pores does not cause any particular problem, the specific surface area is preferably 150 $m^2$/g or less and more preferably 120 $m^2$/g or less.

The porosity of the support is preferably 40% or more and 90% or less. When the porosity is 40% or more, a region sufficient for the adsorption of extracellular vesicles can be ensured. When the porosity is 90% or less, the strength of the support can be sufficiently ensured. The porosity is more preferably 45% or more and 90% or less.

When the support of the present invention is used for separating and purifying extracellular vesicles, the zeta potential, the specific surface area, the porosity and the like are preferably adjusted within the specific range. Exosomes are extracellular vesicles with the diameter in the range of 30 nm to 150 nm and has negative charge on the surface. For example, when a pore is too small and the number of micropores is too large in the case where an exosome in a biological sample such as a cell culture supernatant is efficiently adsorbed and desorbed, an exosomes cannot enter the pores; as a result, even when the specific surface area of the support is increased, an adsorption region and an adsorption capacity may not be sufficiently ensured. When the pores or the number of macropores are too large, the adsorption efficiency of the target exosome may be decreased in some cases, since electrostatic attractive force becomes less effective due to the long distance between the membrane surface and the exosome or extracellular vesicles having the diameter of more than 150 nm enters into the pores. On the one hand, the ion-exchange membrane may preferably have a certain amount of micropores in some cases. A biological sample and a cell culture supernatant contain a large variety of low-molecular-weight substances, many of which carry electric charges. Adsorption sites in mesopores and macropores can be effectively utilized for adsorbing an exosome by adsorbing such low-molecular-weight substances within micropores. The specific surface area of the pores having the pore diameter of less than 30 nm is preferably 30 $m^2$/g or more and 80 $m^2$/g or less.

The ion exchange capacity of the positively charged support is preferably 0.08 meq/g or more and 0.3 meq/g or less. When the ion exchange capacity is 0.3 meq/g or less, the strength of the support can be sufficiently ensured in water, since the content of a positively charged group per one polymer molecule constituting the support is not excessively high and the water solubility of the support is not excessively increased. When the ion exchange capacity is 0.08 meq/g or more,

the high adsorption efficiency of extracellular vesicles can be maintained more reliably. The positively charged group is preferably a tertiary amine group or a quaternary ammonium group. Diethylamine can be used as the tertiary amine, and trimethylammonium can be used as the quaternary ammonium.

The term "contact" means that the support is immersed in a biological sample in the present invention. When the support has a porous structure, the term "contact" also includes the case that a pressure difference is applied between a primary side and a secondary side of the membrane, whereby the biological sample is caused to pass through the interior of the porous membrane from the primary side to the secondary side in order to allow a biological sample to enter the interior of the porous membrane. In addition, when the support is a resin particle, the term "contact" also includes the case that a biological sample is caused to pass from an inlet to an outlet of a column filled with the resin by applying a pressure difference.

The amount of an extracellular vesicle particle that can be treated with the support is dependent on the amount of a residual medium component in a culture supernatant. When the anion exchange capacity of the support is 0.08 to 0.3 meq/g, the particle amount is approximately $10^9$ to $10^{12}$ particles/mL. It is needed to adjust the amount of the biological sample relative to the amount of the support so as not to exceed that range.

4. Washing step

Extracellular vesicles or the like are adsorbed on the positively charged support by contacting a biological sample with the support, and then the support is preferably washed with a buffer solution in the present invention. The buffer solution is not particularly restricted and is preferably Tris-HCl. An example of the other buffer solution includes TBS and PBS. The salt concentration of the buffer solution is preferably adjusted to the salt concentration of a biological sample or less to prevent the target extracellular vesicle from desorbing from the support and eluting together with the impurity. Specifically, the salt concentration is preferably 50 mmol/L or more and 150 mmol/L or less and 6.08 mS/cm or more and 16.01 mS/cm or less. The pH is not particularly restricted and is preferably adjusted to 7 to 8. The amount of the buffer solution for washing is preferably includes within the range of 5 times to 100 times of the volume of the column or the porous body.

5. Recovery step

A buffer solution containing a metal salt is contacted with the support on which extracellular vesicles has been adsorbed in the above-described Step 3 and the Step 4 to desorb the extracellular vesicle to be recovered from the support in this step. A buffer solution containing a monovalent or divalent metal salt may be contacted to elute and recover extracellular vesicles such as an exosome adsorbed on and bound to the support in the present invention. The salt concentration of the buffer solution is preferably 300 mmol/L or more and 800 mmol/L or less and 30.91 mS/cm or more and 80.58 mS/cm or less. The buffer solution may be preferably contacted with increasing the salt concentration in some cases by a stepwise elution method or a linear gradient elution method depending on the purpose such as recovering extracellular vesicles separately by the type based on differences in their charges. A monovalent or divalent metal salt is not particularly restricted and is preferably NaCl. The metal salt may be $MgCl_2$, $Na_2SO_4$ or $MgSO_4$ as the other embodiments. The buffer solution is preferably Tris-HCl, PBS and TBS and more preferably Tris-HCl.

The salt concentration and the electrical conductivity of a sample containing extracellular vesicles may be adjusted to improve the storage stability of the recovered extracellular vesicle in the present invention. The salt concentration and the electrical conductivity may be adjusted within the concentration range close to a physiological electrolyte concentration and are preferably 140 mmol/L or more and 160 mmol/L or less and more than 15 mS/cm and 17.01 mS/cm or less. The electrical conductivity is more preferably 15.01 mS/cm or more. An example of a method for measuring the salt concentration includes a method in which the electrical conductivity of the recovered fraction is measured to calculate an NaCl concentration (Figure 2). The fraction may be diluted by adding 10 mmol/L Tris-HCl (pH7.5) depending on the measured salt concentration. Alternatively, the buffer solution may be substituted by ultrafiltration or the fraction may be desalinated by dialysis.

The term "concentrate" means that the volume of a solution containing the target extracellular vesicle is decreased to increase the concentration of the target extracellular vesicle particle in the present invention.

The present application claims the benefit of the priority date of Japanese patent application No. 2023-149357 filed on September 14, 2023. All of the contents of the Japanese patent application No. 2023-149357 filed on September 14, 2023, are incorporated by reference herein.

EXAMPLES

The present invention is hereinafter described in more detail with reference to Examples and Comparative Examples, and the present invention is not restricted to the following Examples. The methods for evaluating physical

properties and the like in the following Examples are described below.

Test example 1: Measurement of ion exchange capacity of support

The ion exchange capacity of the support was measured as follows.

A 0.1 mass% bromophenol blue (BPB) aqueous solution was prepared, and the positively charged support was immersed therein at room temperature for 1 hour to be stained. The stained support was washed with water and then immersed in 8 mass% sodium chloride aqueous solution to elute the BPB adsorbed on the support. The absorbance at 590 nm of the sodium chloride aqueous solution was measured, and the number of moles of BPB adsorbed per unit mass of the support was calculated as an ion exchange capacity on the basis of a calibration curve prepared using a BPB aqueous solution containing 8 mass% sodium chloride.

Test example 2: Evaluation of particle rejection rate

The rejection rates of the porous substrate membrane and the ion-exchange membrane were evaluated using a polystyrene particle dispersion. A Tween 20 aqueous solution prepared by adding and dispersing various sizes of polystyrene particles at 0.01 mass% was passed through the membrane at an operating pressure of 10 kPa to obtain a permeate. Polystyrene particle concentrations in the original solution and the permeate were measured on the basis of the absorbance at 250 nm, and the rejection rate was calculated according to the following formula.

$$\text{Rejection rate (\%)} = \{1-(Ca-Cb)/Ca\}\times 100$$

Ca: polystyrene particle concentration in the original solution
Cb: polystyrene particle concentration in the permeate

The rejection rate was measured using polystyrene having the particle diameter of 79, 132, 208, 262, 313, 420, 460 and 616 nm, and the polystyrene particle diameter at which the rejection rate reached 80% was defined as the minimum pore diameter.

Test example 3: Observation of membrane structure

The membrane to be observed was placed on an SEM sample stage, dried at room temperature, coated with platinum, and observed using a scanning electron microscope ("SU-1500" manufactured by Hitachi High-Technologies).

Test example 4: Measurement of average pore diameter on large-pore side of membrane

The average pore diameter on the large-pore side was measured using the SEM image of the membrane surface observed at a magnification of 10,000× by the following procedure. The image was opened in the image processing software, "Image J", a line was drawn over the scale bar using the straight line tool from the toolbar, and Analyze > Set Scale was selected. The length of the scale bar was entered as the "Known Distance", and the unit was entered as the "Unit of Length". The image was binarized by selecting Image > Adjust > Auto Threshold, setting the mode of Method to Default, and clicking OK. It was confirmed that "Feret's diameter" was checked, and OK was pressed in Analyze > Set Measurements. In Analyze > Analyze Particles, the "Size (pixel^2)" was set as 0-Infinity, it was confirmed that the checkboxes for "Display results", "Exclude on edges" and "Include holes" were selected, and OK was pressed to obtain the Feret diameter measurement data in the Results window. The length corresponding to 15 pixels was calculated in the same unit as the Feret diameter and the values below this threshold were discarded to remove noise of 15 pixels or less, and the average Feret diameter was calculated.

Test example 5: Measurement of zeta potential

The zeta potential of the membrane was determined using a Zetasizer Nano ZS equipped with a flat-cell for zeta potential measurement ("ZEN1020" manufactured by Malvern Panalytical). The mobility of aluminum oxide particles was measured in ion-exchanged water at pH 6.5 to 7.5 to calculate the zeta potential.

Test example 6: Measurement of specific surface area

The specific surface area was measured using a pore size distribution analyzer ("Autopore IV 9520" manufactured Shimadzu Corporation) under the following conditions. The sample was cut into strips of approximately 12.5 mm × 25 mm, and 0.04 to 0.15 g was added into a 5 mL powder cell (stem volume: 0.4 mL). Measurements were performed at an initial pressure of approximately 3.7 kPa corresponding to a pore diameter of about 340 μm. The mercury contact angle of the device defaults was set to 130° and a mercury surface tension was set to 485 dynes/cm with respect to mercury parameters. The pore size distribution curve was obtained by plotting the logarithmic differential volume of mercury intruded into the sample against the pore diameter. The total specific surface area was determined by measuring the pore volume V corresponding to each mode diameter D, calculating the specific surface area S for each mode diameter D using the following formula, and summing the specific surface areas over the range of 0.03 to 500 μm.

$$S = 4V/D$$

Test example 7: Nanoparticle tracking analysis

The average particle number and the diameter of the recovered extracellular vesicle were measured using a nanoparticle analysis system ("NanoSight NS300" manufactured by Malvern Panalytical) by nanoparticle tracking analysis (NTA). The camera level was set to 14 and the detection threshold to 7. When the salt concentration of the culture supernatant was changed, the particle number was measured after 24 hours or more had elapsed following the change.

Test example 8: Western blotting

Western blotting was performed to detect extracellular vesicle proteins such as a surface protein in the recovered extracellular vesicle. The sample containing the recovered extracellular vesicle was concentrated as necessary, dissolved in a sample buffer ("NuPage LDS Sample Buffer" manufactured by Thermo Fisher Scientific), and boiled at 95°C for 5 minutes. A sample buffer additionally containing 100 mmol/L dithiothreitol was used for the detection of TSG101, β-actin and GAPDH, and a sample buffer without dithiothreitol was used for the detection of CD63 and CD81. Each sample and molecular weight marker ("MagicMark XP Western Protein Standard" manufactured by Thermo Fisher Scientific) were separated by electrophoresis on a 10% polyacrylamide gel using SDS running buffer (Tris/Glycine/SDS Buffer) at 30 mA for a single gel or 40 mA for two gels for 60 minutes. The resulting gel was transcribed onto a polyvinylidene fluoride membrane using a blotting buffer ("EzFastBlot" manufactured by ATTO), blocked with a blocking reagent ("Blocking One" manufactured by Nacalai Tesque), and then incubated with the respective primary antibody for 1 hour. The membrane was incubated with the respective secondary antibody for 1 hour after washing. The target protein bands were visualized using an HRP detection reagent ("EzWestLumi Plus" manufactured by ATTO) after washing.

Example 1

(1) Step to prepare biological sample

Human embryonic kidney-derived cell line (HEK293.2sus) was used as the culture cell. HEK293.2sus was obtained from the American Type Culture Collection. For culturing HEK293.2sus cell, 293 SFM II medium (Thermo Fisher Scientific) was used. The culture was carried out in the conditions of 100 rpm, 8% $CO_2$ and a cell density of $5 \times 10^5$ cells/mL in a 100 mL Erlenmeyer flask (Corning). Subsequently, the cell was cultured in a 70 $cm^2$ flask (Corning) using 293 SFM II medium under 5% $CO_2$. The culture medium was replaced every 3 or 4 days, and the cell was cultured for a total of 17 days. The culture medium collected at each medium change was pooled and used for exosome preparation. The collected medium was centrifuged at 4°C, first at 300 × g for 10 minutes, followed by 10,000 × g for 20 minutes, and the supernatant was collected. The culture supernatant was further filtered through a 0.45 μm filter (Corning) to prepare the final culture supernatant.

(2) Step to prepare anion-exchange membrane

For a positively charged support, a cellulose acetate membrane filter (ADVANTEC) with a nominal pore size of 0.2 μm was deacetylated by immersing the membrane filter in 0.1 M sodium hydroxide aqueous solution at room temperature for 4 hours. The resulting deacetylated membrane was then immersed in 0.4 M glycidyl diethylamine aqueous solution at 65°C for 4 hours to obtain an ion-exchange membrane. The performance of the resulting ion-exchange membrane is shown in Table 1.

Table 1

| | |
|---|---|
| Ion exchange capacity | 0.1 meq/g |
| Zeta potential | 35 mV |
| Specific surface area of pores of 30 nm or more | 9.6 $m^2$/g |
| Minimum pore diameter | 0.35 μm |

(3) Equilibration step

A 25 mm-diameter anion-exchange membrane was equilibrated by passing 5 mL of 10 mmol/L Tris-HCl (pH 7.5) containing 100 mmol/L NaCl (11.05 mS/cm) as an equilibration buffer through the membrane.

(4) Permeation step

NaCl was dissolved in 10 mmol/L Tris-HCl buffer, and a calibration curve showing the relationship between NaCl concentration and electrical conductivity was prepared. Electrical conductivity was measured using an electrical conductivity meter ("LAQUAtwin EC-33" manufactured by HORIBA). The measurement cell was rinsed twice with the sample to be measured prior to the measurement. The culture supernatant (6 mL) filtered through a 0.45 μm membrane was mixed with 3 mL of 10 mmol/L Tris-HCl buffer to adjust the electrical conductivity to 11.05 mS/cm and passed through the membrane.

(5) Elution step

The membrane was washed by passing 10 mL of 10 mmol/L Tris-HCl buffer through the membrane, and then 2 mL of 300 mmol/L NaCl (30.91 mS/cm) was passed through the membrane to elute the extracellular vesicle adsorbed on the membrane, thereby obtaining the purified fraction of extracellular vesicle.

(6) Isotonicization step

Next, the electrical conductivity of the fraction into which the extracellular vesicle had been eluted was measured. The NaCl concentration was calculated from the electrical conductivity value using the calibration curve (Figure 2), and then 10 mmol/L Tris-HCl (pH 7.5) was immediately added to adjust the solution to isotonic condition of 10 mmol/L Tris-HCl (pH 7.5) containing 0.15 mol/L NaCl as the extracellular vesicle solution. The step for equilibration to the step for isotonicization were all performed with the solutions cooled to 4 to 10°C.

(7) Characterization

The fractionated sample containing extracellular vesicles was analyzed by NTA to measure the average particle number and the diameter, and the presence of extracellular vesicles was confirmed by Western blotting for detecting CD63. NTA analysis was performed using a nanoparticle analysis system ("NanoSight NS300" manufactured by Malvern Panalytical) with a camera level of 14 and a detection threshold (DT) of 7. The detection was carried out by Western blotting using anti-CD63 mouse antibody (MEX002-3, MBL) as the primary antibody, Anti-Mouse IgG HRP-Linked Whole Ab Sheep (Cytiva) as the secondary antibody and an iBright™ FL1500 Imaging System (Thermo Fisher Scientific).

Comparative example 1

An extracellular vesicle solution was prepared similarly to Example 1 except that the electrical conductivity of the culture supernatant was not adjusted and the culture supernatant with the original salt concentration was passed through the membrane in the permeation step. The electrical conductivity of the culture supernatant that permeated the anion-exchange membrane was 16.01 mS/cm at the time.

Comparative example 2

An extracellular vesicle solution was prepared similarly to Example 1 except that an equilibration buffer was added to the culture supernatant to adjust the electrical conductivity to 3.10 mS/cm and then the culture supernatant was passed through the membrane in the permeation step.

Table 2

| | Example 1 | Comparative example 1 | Comparative example 2 |
|---|---|---|---|
| Electrical conductivity of culture supernatant [mS/cm] | 11.03 | 16.01 | 3.10 |
| Particle number per 1 mL after adjusting salt concentration in culture supernatant (after 72 hours) [10^8 particles] | 16.2±0.4 | 19.5±2.0 | 29.2±0.7 |
| Salt concentration of elution buffer [mol/L] | 0.5 | 0.5 | - |
| Recovered particle number [10^8 particles] | 113 | 93 | - |

As shown in the results in Table 2, the number of the particles recovered using the support having an ion-exchange group was decreased in Comparative Example 1, in which the culture supernatant having the electrical conductivity of 16.01 mS/cm without adjusting the electrical conductivity was subjected to purification. This result indicates that extracellular vesicles was not sufficiently adsorbed onto the support and instead passed through in the flow-through fraction due to the high salt concentration of the culture supernatant.

The number of the particles in the culture supernatant measured by NTA was significantly increased in Comparative Example 2, in which the electrical conductivity of the culture supernatant was adjusted to 3.10 mS/cm. This result indicates that the extracellular vesicle was ruptured and damaged. Since the significant alteration of the extracellular vesicle was observed in Comparative Example 2, the permeation test was not performed.

The result of the Western blotting test is shown in Figure 3. CD63 is known as a marker for extracellular vesicles (EVs) and has been reported to be particularly expressed in an exosome among EVs (Borges FT, et al., 2013, Extracellular vesicles: structure, function, and potential clinical uses in renal diseases, Braz J Med Biol Res, 46(10), 824-30). In addition, it has been reported that an exosome-rich fraction exhibits weaker negative charge in comparison with other EVs such as a microvesicle (Seo N, et al., 2022, Distinguishing functional exosomes and other extracellular vesicles as a nucleic acid cargo by the anion-exchange method, J Extracell Vesicles, Mar, 11(3)). The proportion of CD63-positive particle per unit of recovered particles in the purified fraction was higher in Example 1 than in Comparative Example 1 as is evident from Figure 3. This result indicates that the membrane can adsorb a greater number of an exosome with particularly weak negative charge among the particles in the culture supernatant and an exosome can be recovered with a reduced loss by using the method disclosed herein.

INDUSTRIAL APPLICABILITY

Extracellular vesicles contained in a biological sample can be easily purified and recovered with a high recovery rate according to the present invention. The extracellular vesicle obtained by the present invention can be suitably used in fields such as drug delivery, pharmaceuticals, in vitro diagnostics, food and cosmetics.

DESCRIPTION OF REFERENCE SIGNS

1, 2: Liquid culture medium reservoir
3: Cell collection container
4, 5, 6, 7, 8, 9, 10, 11, 12: Valve
13, 14, 15, 16: Aseptic connector
17: Cell culture vessel
18: Waste liquid collection container
19, 20: Liquid feed pump
21: Operation controller (Operation panel)
31: $CO_2$ incubator

**Claims**

**1.** A method for producing extracellular vesicles, the method comprising:

Step (a) of preparing a biological sample comprising the extracellular vesicles,

Step (b) of adjusting an electrical conductivity of the biological sample to 4 mS/cm or more and 15 mS/cm or less,
Step (c) of contacting the biological sample with a positively charged support, and
Step (d) of collecting the extracellular vesicles by contacting the support with a buffer solution comprising a metal salt.

2. The method according to claim 1, wherein a cell is isolated from an animal or a frozen cell from a cell stock is thawed, and subsequently the cell or the thawed cell is cultured in the Step (a).

3. The method according to claim 1, comprising a step of removing all or a part of an impurity from the biological sample between the Step (a) and the Step (b).

4. The method according to claim 3, wherein 1 or more selected from the group consisting of centrifugation, precision filtration, ultrafiltration and dialysis are carried out in the step of removing all or a part of the impurity.

5. The method according to claim 1, wherein the support is an anion-exchange separation membrane in the Step (c).

6. The method according to claim 5, wherein the support has a tertiary amine and/or a quaternary ammonium group in the Step (c).

7. The method according to claim 1, comprising a step of washing the support by contacting the support with a buffer solution between the Step (c) and the Step (d).

8. The method according to claim 1, wherein the buffer solution comprising the metal salt is any one of Tris-HCl buffer solution, phosphate-buffered saline solution and Tris-buffered saline solution in the Step (d).

9. The method according to claim 1, wherein the metal salt is NaCl in the Step (d).

10. The method according to claim 1, wherein an electrical conductivity of a sample comprising the collected extracellular vesicles is adjusted to more than 15 mS/cm in the Step (d).

11. The method according to claim 1, wherein the extracellular vesicle is exosomes and/or microvesicles.

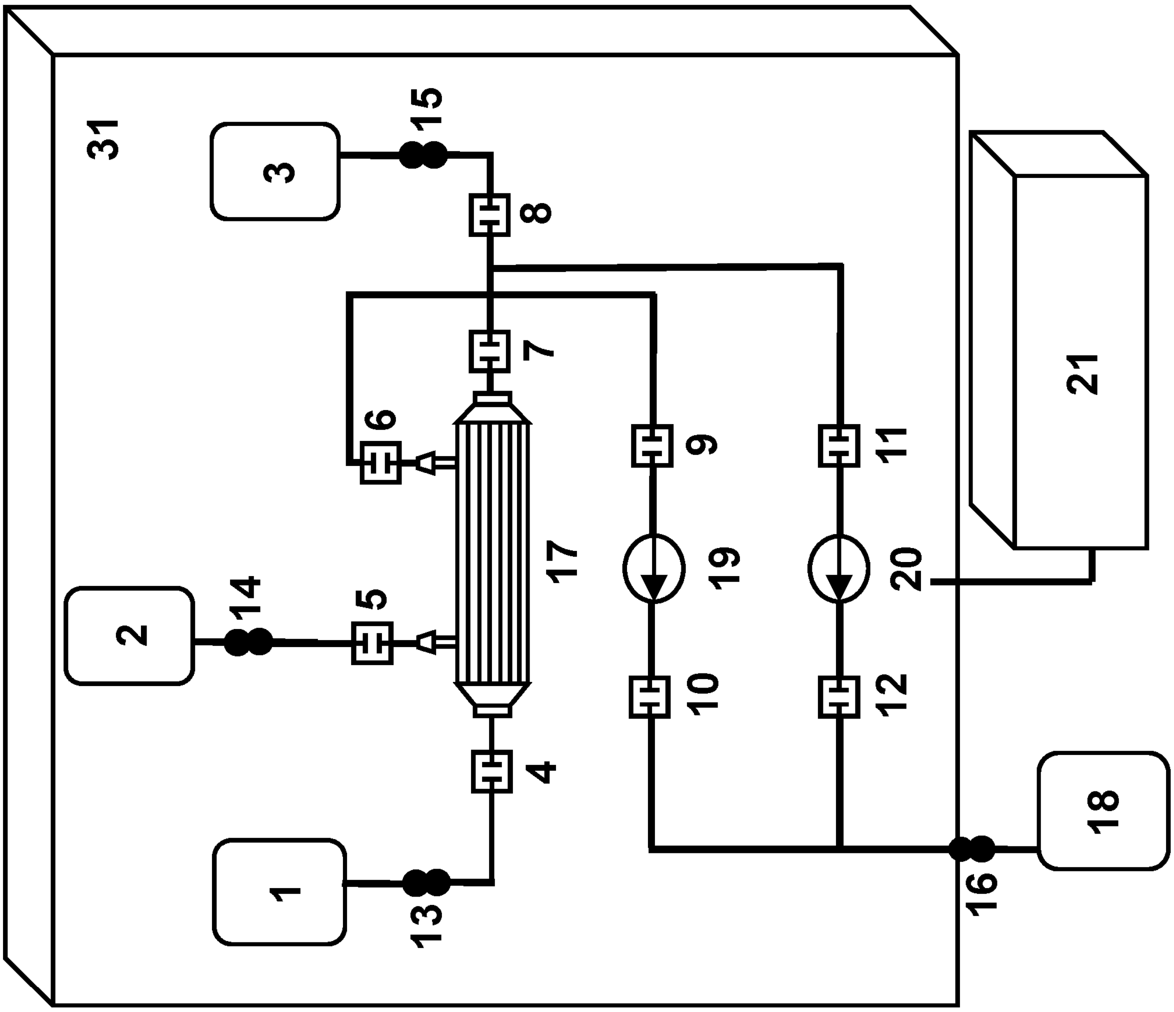


[Figure 1]

[Figure 2]

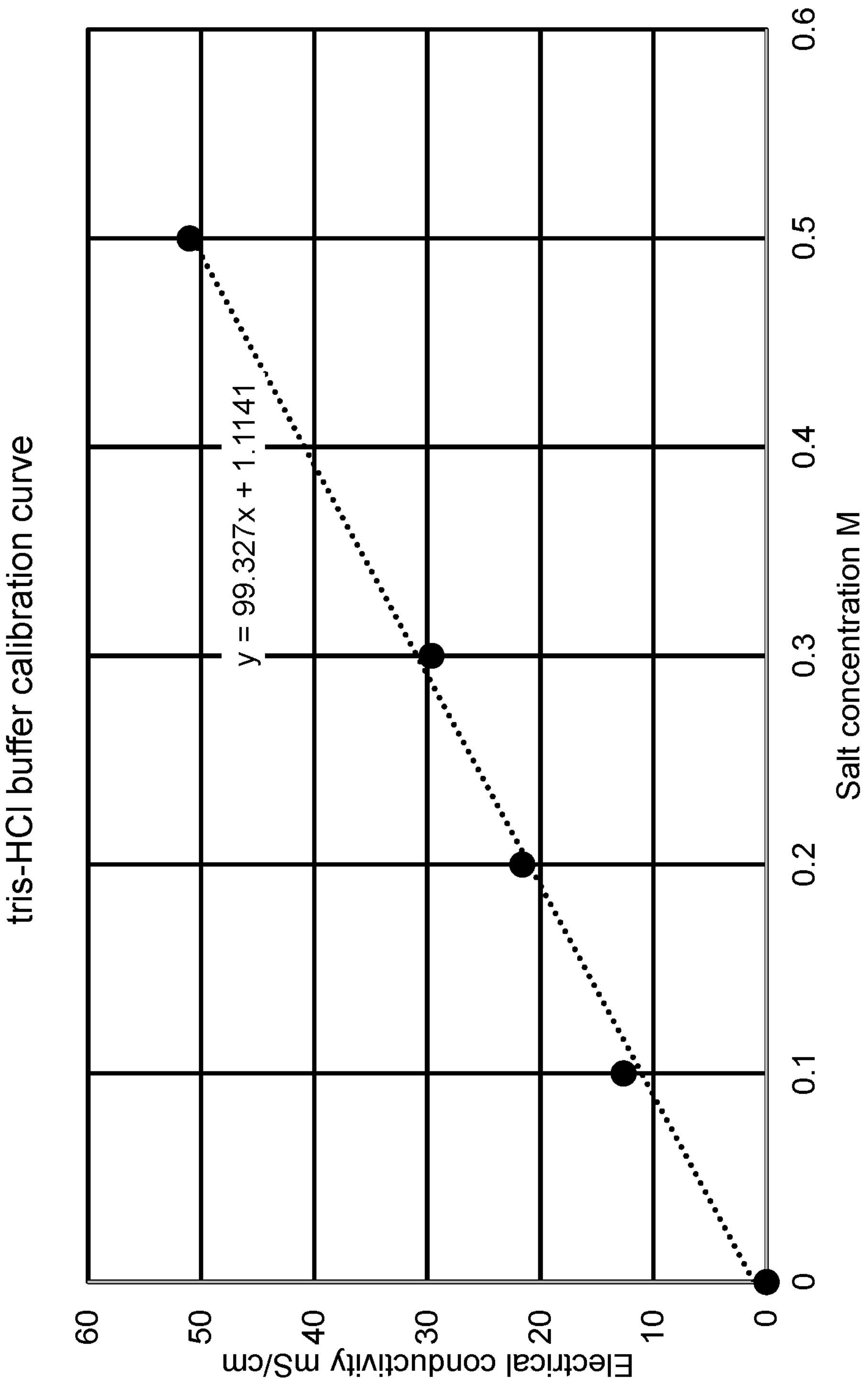
tris-HCl buffer calibration curve
y = 99.327x + 1.1141
Electrical conductivity mS/cm
0
10
20
30
40
50
60
Salt concentration M
0
0.1
0.2
0.3
0.4
0.5
0.6

[Figure 3]

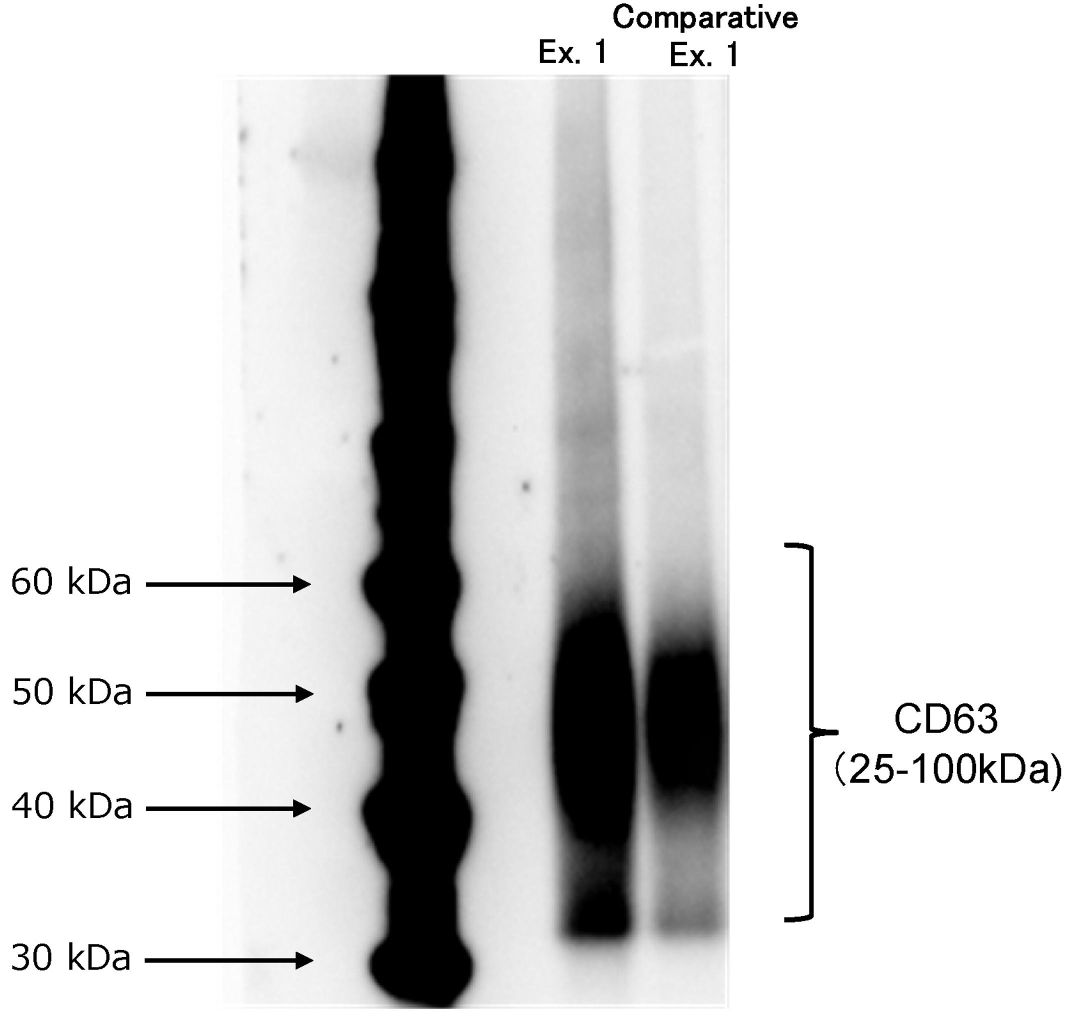


Primary antibody: Anti CD63(LAMP-3), Monoclonal Antibody
Secondary antibody: Anti-Mouse IgG, HRP -Linked Whole Ab Sheep
3×10^8 particles/test

**INTERNATIONAL SEARCH REPORT**

International application No.
**PCT/JP2024/031255**

**A. CLASSIFICATION OF SUBJECT MATTER**

***C12N 5/071***(2010.01)i
FI: C12N5/071

According to International Patent Classification (IPC) or to both national classification and IPC

**B. FIELDS SEARCHED**

Minimum documentation searched (classification system followed by classification symbols)

C12N5/071

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2012/087241 A1 (AGENCY FOR SCIENCE, TECHNOLOGY AND RESEARCH) 28 June 2012 (2012-06-28)<br>claim 1, p. 10, lines 3-5, p. 11, lines 23-24, p. 12, lines 2-3, p. 13, lines 8-23, p. 15, lines 6-8, p. 15, line 23 to p. 16, line 7, p. 36, lines 11-15, p. 15, line 23 to p. 16, line 7, p. 37, lines 6-7, p. 38, lines 9-15 | 1, 3-9, 11 |
| Y | claim 1, p. 10, lines 3-5, p. 11, lines 23-24, p. 12, lines 2-3, p. 13, lines 8-23, p. 15, lines 6-8, p. 15, line 23 to p. 16, line 7, p. 36, lines 11-15, p. 15, line 23 to p. 16, line 7, p. 37, lines 6-7, p. 38, lines 9-15 | 1-11 |
| X | WO 2020/027185 A1 (UNIV MIE) 06 February 2020 (2020-02-06)<br>paragraphs [0031]-[0032], [0036]-[0037] | 1-4, 7, 9-11 |
| Y | paragraphs [0031]-[0032], [0036]-[0037], [0041] | 1-11 |
| Y | JP 2016-502862 A (EXOSOME DIAGNOSTICS INC.) 01 February 2016 (2016-02-01)<br>claims 18-24, 30, paragraph [0015] | 1-11 |

☑ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/031255**

**C. DOCUMENTS CONSIDERED TO BE RELEVANT**

| Category* | Citation of document, with indication, where appropriate, of the relevant passages | Relevant to claim No. |
|---|---|---|
| A | 高野　清, マイクロ流動場の制御による均一巨大単層リポソームの高効率生成法の開発, [online], 25 September 2014, KAKENHI, [retrieved on 06 November 2024], Internet<URL: https://kaken.nii.ac.jp/ja/file/KAKENHI-PROJECT-23656145/23656145seika.pdf>, (TAKANO, Kiyoshi, Development of highly-efficient process for producing giant unilamellar vesicles (GUV) by controlling microscopic flow field)<br>p. 3, right column, lines 33-34 | 1-11 |

Form PCT/ISA/210 (second sheet) (July 2022)

**INTERNATIONAL SEARCH REPORT**
**Information on patent family members**

International application No.
**PCT/JP2024/031255**

| Patent document cited in search report | Publication date (day/month/year) | Patent family member(s) | Publication date (day/month/year) |
|---|---|---|---|
| WO 2012/087241 A1 | 28 June 2012 | US 2014/0004601 A1 | |
| WO 2020/027185 A1 | 06 February 2020 | US 2021/0239664 A1 paragraphs [0108], [0110], [0116], [0118], [0124] | |
| JP 2016-502862 A | 01 February 2016 | US 2015/0353920 A1 claims 18-24, 30, paragraph [0015] | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020027185 A
- JP 2023149357 A


### Non-patent literature cited in the description


- **BORGES FT et al.** Extracellular vesicles: structure, function, and potential clinical uses in renal diseases. *Braz J Med Biol Res*, 2013, vol. 46 (10), 824-30
- **SEO N et al.** Distinguishing functional exosomes and other extracellular vesicles as a nucleic acid cargo by the anion-exchange method. *J Extracell Vesicles*, 2022, vol. 11 (3)